# EUROPÄISCHE PATENTSCHRIFT

(11) **EP 0 775 478 B1**
(45) Veröffentlichungstag und Bekanntmachung des Hinweises auf die Patenterteilung: **13.02.2002**
(21) Anmeldenummer: 96117992.6
(22) Anmeldetag: 09.11.1996
(51) Int. Cl.: A61K 7/06, A61K 7/48, A61K 7/32

(54) **Gegen Bakterien, Mycota und Viren wirksame Wirkstoffkombinationen auf der Basis von Partialglyceriden und dialkylsubstituierten Essigsäuren**
Combinations of active ingredients based on partial glycerides and dialkylsubstituted acetic acids efficient against bacteria, mycoses and viruses
Combinaisons d'agents actifs à base de glycérides partiels et d'acide acétique dialkylsubstitués

(30) Priorität: 23.11.1995 DE 19543696
(43) Veröffentlichungstag der Anmeldung: 28.05.1997
(73) Patentinhaber: Beiersdorf Aktiengesellschaft, 20245 Hamburg (DE)
(72) Erfinder: Hoppe, Udo, Dr., 22397 Hamburg (DE); Wolf, Florian, Dr., 20251 Hamburg (DE); Traupe, Bernd, 22457 Hamburg (DE); Schreiber, Jörg, Dr., D-22087 Hamburg (DE)

(56) Entgegenhaltungen:
- EP-A- 0 013 755
- EP-A- 0 588 379
- EP-A- 0 697 213
- DE-A- 2 339 149
- DATABASE WPI Week 9542 Derwent Publications Ltd., London, GB; AN 95-325458 XP002026084 "Cosmetic material for moisturising lips - comprises oil contg. ester of diglycerine and 6-18C branched monocarboxylic acid, and 6-10C dicarboxylic acid" & JP 07 223 925 A (NOEVIR) , 22.August 1995
- BLOCK, SEYMOUR S.: "Disinfection, Sterilization, and Preservation", 1991, LEA & FEBIGER, USA

## Beschreibung

Die vorliegende Erfindung betrifft die Verwendung an sich bekannter Substanzen als gegen Bakterien und Mycota wirksame Substanzen. In besonderen Ausführungsformen betrifft die vorliegende Erfindung kosmetische und dermatologische Zubereitungen, solche Substanzen enthaltend.

Der gesunde warmblütige Organismus, insbesondere die gesunde menschliche Haut, ist mit einer Vielzahl nichtpathogener Mikroorganismen besiedelt Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.
Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi [fungus = lat. Pilz], Mycota [µυκη 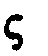 = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kemhülle und Kemmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung vonPityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Femer sind Superinfektionen der Haut durch Pilze und Bakterien nicht selten.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußem. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche. aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Es wurde überraschenderweise gefunden, und darin liegt die Lösung all dieser Aufgaben, daß Verwendung von Wirkstoffkombinationen, bestehend aus
(I) einer oder mehrerer Substanzen, gewählt aus der Gruppe der Monoglycerin-monocarbonsäureester, der Diglycerin-monocarbonsäureester, der Triglycerin-monocarbonsäureester, der Monoglycerin-dicarbonsäure-monoester, der Diglycerin-dicarbonsäuremonoester und der Triglycerin-dicarbonsäure-monoester sowie
(II) einer oder mehreren dialkylsubstituierten Essigsäuren der Formel 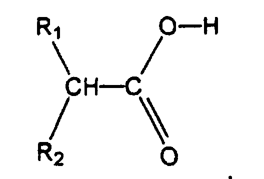 wobei R1 einen verzweigten oder unverzweigten Alkylrest mit 1 - 12 Kohlenstoffatomen
   und R2 einen verzweigten oder unverzweigten Alkylrest mit 1 - 24 Kohlenstoffatomen darstellt,
zur Herstellung kosmetischer oder dermatoligischer Zubereitungen zur Bekämpfung von Körpergerüchen zur Bekämpfung von Kopfschuppen.
den Nachteilen des Standes der Technik abhelfen.

Zwar beschreiben die EP-A-0 013 755 und Block, Seymour, "Disinfection, Sterilization and Preservation", 1991, Lea & Febiger, USA, bestimmte Zubereitungen, die auch verzweigte Carbonsäuren umfassen können, der Weg zur vorliegenden Erfindung konnte jedoch dadurch nicht vorgezeigt werden.

Vorteilhaft werden die Alkylreste der dialkylsubstituierter Essigsäuren so gewählt daß R₁ = Methyl, Ethyl, Propyl, Butyl, Pentyl oder Hexyl darstellt.

Weiter vorteilhaft werden die Alkylreste so gewählt, daß R₂= Octyl, Nonyl, Decyl, Undecyl, Dodecyl darstellt.

Insbesondere vorteilhaft ist, die erfindungsgmäßen Essigsäurederivate aus der Gruppe 2-Butyloctansäure, 2-Butyldecansäure, 2-Hexyloctansäure, 2-Hexyldecansäure zu wählen.

Ganz besonders bevorzugt ist die 2-Butyloctansäure.

Es hat sich in erstaunlicher Weise herausgestellt, daß die erfindungsgemäßen Wirkstoffkombinationen das Wachstum von grampositiven und gramnegativen Bakterien sowie Mycobionten verhindern.

Insbesondere sind die erfindungsgemäßen Wirkstoffkombinationen befähigt, daß Wachstum von Hefen, insbesondere der Pityrosporum-Arten, namentlich Pityrosporum ovale, zu verhindern.

Es hat sich femer herausgestellt, daß die erfindungsgemäßen Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, zu beseitigen.

Die erfindungsgemäßen Wirkstoffkombinationen eignen sich darüberhinaus gut für die Verwendung als desodorierender Wirkstoff in kosmetischen Desodorantien sowie gegen unreine Haut, leichte Formen der Akne bzw. Propionibakterium acnes.

Der Stand der Technik lieferte folglich nicht den geringsten Hinweis auf die erfindungsgemäße Verwendung als antimycotisches Wirkprinzip.

Femer war erstaunlich, daß die erfindungsgemäßen Wirkstoffkombinationen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Antischuppenshampoos.

Erfindungsgemäß werden die dialkylsubstituierten Essigsäuren bevorzugt in kosmetischen oder dermatologischen Zusammensetzungen eingesetzt einem Gehalt von 0,005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an den erfindungsgemäßen dialkylsubstituierten Essigsäuren, ganz besonders vorteilhaft 0,5 - 3,0 Gew.-%, jeweils bezogen auf das Gesamtgewicht der Zusammensetzung.

Es ist auch von Vorteil, anstatt reiner erfindungsgemäßer dialkylsubstituierter Essigsäuren solche Stoffe zu verwenden, welche sich ihrerseits durch einen Gehalt an erfindungsgemäßen dialkylsubstituierten Essigsäuren auszeichen.

Die erfindungsgemäßen Monoglycerin-mono- bzw. -dicarbonsäure-monoester werden durch die allgemeine Formel 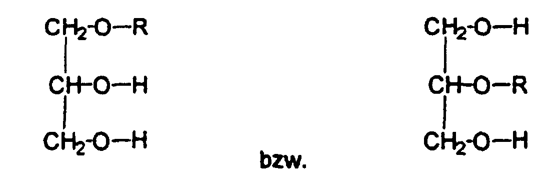 wiedergegeben, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estem zugrundeliegenden Fettsäuren bzw. Monocarbonsäuren sind die

| | | |
|---|---|---|
| Hexansäure | (Capronsäure) | (R = -C(O)-C₅H₁₁), |
| Heptansäure | (Önanthsäure) | (R = -C(O)-C₆H₁₃), |
| Octansäure | (Caprylsäure) | (R = -C(O)-C₇H₁₅), |
| Nonansäure | (Pelargonsäure) | (R = -C(O)-C₈H₁₇), |
| Decansäure | (Caprinsäure) | (R = -C(O)-C₉H₁₉), |
| Undecansäure | | (R = -C(O)-C₁₀H₂₁), |
| Undecensäure | | (R = -C(O)C₁₀H₁₉), |
| Dodecansäure | (Laurinsäure) | (R = -C(O)-C₁₁H₂₃), |
| Tridecansäure | | (R = -C(O)-C₁₂H₂₅), |
| Tetradecansäure | (Myristinsäure) | (R = -C(O)-C₁₃H₂₇). |

Besonders vorteilhaft stellt R den Octanoylrest (Caprylsäurerest) bzw. den Decanoylrest (Caprinsäurerest) dar, wird also also durch die Formeln

R = -C(O)-C₇H₁₅) bzw. R = -C(O)-C₉H₁₉

repräsentiert.

In dieser Schrift, insbesondere in den Beispielen, werden folgende Kürzel verwendet: GMCy für Glycerinmonocaprylat,
GMC für Glycerinmonocaprinat,
GMS für Glycerinmonostearat,
GMU für Glycerinmonoundecylat.

Bei den in 1-Position des Glycerins veresterten Glycerinestern ist die 2-Position ein Asymmetriezentrum. Erfindungsgemäß aktiv und gleichermaßen von Vorteil sind die 2S- und die 2R-Konfiguration.

Es hat sich als günstig herausgestellt, racemische Gemische der Stereoisomeren zu verwenden.

In den dermatologischen Zubereitungen beträgt der Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Erfindungsgemäß liegen die Di- bzw. Triglycerineinheiten der erfindungsgemäßen Diglycerin-mono- bzw. -dicarbonsäure-monoester bzw. Triglycerin-mono- bzw. -dicarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Mono- bzw. Dicarbonsäuremonoester des Diglycerins sind bevorzugt durch folgende Struktur gekennzeichnet (Substitutionspositionen angegeben): 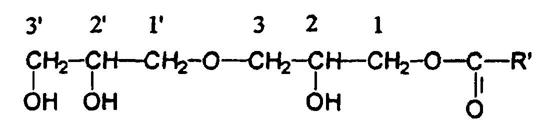 wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Mono- bzw. Dicarbonsäuremonoester des Triglycerins sind bevorzugt durch folgende Struktur gekennzeichnet (Substitutionspositionen angegeben): 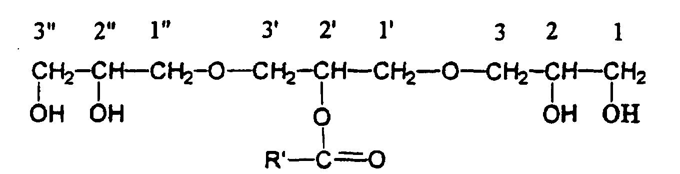 wobei R" einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Monocarbonsäuren sind die

| | | |
|---|---|---|
| Hexansäure | (Capronsäure) | (R' bzw. R''= -C₅H₁₁), |
| Heptansäure | (Önanthsäure) | (R' bzw. R''= -C₆H₁₃), |
| Octansäure | (Caprylsäure) | (R' bzw. R''= -C₇H₁₅), |
| Nonansäure | (Pelargonsäure) | (R' bzw. R''= -C₈H₁₇), |
| Decansäure | (Caprinsäure) | (R' bzw. R''= -C₉H₁₉), |
| Undecansäure | | (R' bzw. R''= -C₁₀H₂₁), |
| 10-Undecensäure | (Undecylensäure) | (R' bzw. R''= -C₁₀H₁₉), |
| Dodecansäure | (Laurinsäure) | (R' bzw. R''= -C₁₁H₂₃), |
| Tridecansäure | | (R' bzw. R''= -C₁₂H₂₅), |
| Tetradecansäure | (Myristinsäure) | (R' bzw. R''= -C₁₃H₂₇), |
| Pentadecansäure | | (R' bzw. R''= -C₁₄H₂₉), |
| Hexadecansäure | (Palmitinsäure) | (R' bzw. R''= -C₁₅H₃₁), |
| Heptadecansäure | (Margarinsäure) | (R' bzw. R''= -C₁₆H₃₃), |
| Octadecansäure | (Stearinsäure) | (R' bzw. R''= -C₁₇H₃₅). |

Besonders günstig werden R' und R" gewählt aus der Gruppe der unverzweigten Alkylreste mit ungeraden C-Zahlen, insbesondere mit 9, 11 und 13 C-Atomen.

Im allgemeinen sind die Monocarbonsäure-monoester des Diglycerins denen des Triglycerins bevorzugt.

Ganz besonders günstig sind

| | | |
|---|---|---|
| Diglycerinmonocaprinat | (DMC) | R' = 9 |
| Triglycerinmonolaurat | (TML) | R'' = 11 |
| Diglycerinmonolaurat | (DML) | R' = 11 |
| Triglycerinmonomyristat | (TMM) | R'' = 13. |

Als bevorzugter erfindungsgemäßer Monocarbonsäuremonoester des Diglycerins hat sich das Diglycerinmonocaprinat (DMC) erwiesen.

Die erfindungsgemäßen Monocarbonsäuremonoester des Diglycerins liegen bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestem des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die den erfindungsgemäßen Estem zugrundeliegenden Dicarbonsäuren werden bevorzugt gewählt aus der Gruppe der α,ω-Alkandicarbonsäuren, insbesondere bevorzugt gewählt aus der Gruppe der α,ω-Alkandicarbonsäuren, so daß die Reste R, R' bzw. R" im gegebenen Falle von der generischen Formel

-OOC-(CH₂)ₖ-COOH

beschrieben werden und wobei k Zahlen von 0 bis 8 annehmen kann.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestem sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2"-Position Asymmetriezentren. Erfindungsgemäß aktiv und gleichermaßen von Vorteil sind die 2S2'S2"S-, die 2R2'S2"S-, die 2S2'R2"S-, die 2R2'R2"S-, die 2S2'S2"R, die 2R2'S2"R-, die 2S2'R2"R- und die 2R2'R2"R-Konfiguration.

Es hat sich als günstig herausgestellt, racemische Gemische der Stereoisomeren zu verwenden.

Es ist von Vorteil, den Gehalt an (i) Mono-, Di- und/oder Triglycerin-mono- bzw. dicarbonsäure-monoester und (ii) Dialkylcarbonsäure bzw. -säuren so zu wählen, daß Verhältnisse (i): (ii) wie 10 : 1 bis 1 : 10, insbesondere wie etwa 5 : ↑ bis ↑ : 5, ganz besonders vorteilhaft wie etwa 2 : 1 bis 1 : 2: 5 entstehen.

Erfindungsgemäße Zubereitungen, die erfindungsgemäßen Wirkstoffkombinationen enthaltend, sind besonders vorteilhaft dadurch gekennzeichnet, daß die erfindungsgemäßen Wirkstoffkombinationen in Konzentrationen von 0,05 - 10,00 Gew.-%, bevorzugt 0,1 - 5,0 Gew.-%, vorliegen, jeweils bezogen auf das Gesamtgewicht der Zubereitungen.

Erfindungsgemäße dermatologische Zubereitungen können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Crèmes oder Lotionen. Weiterhin können die Zubereitungen vorteilhaft in Form von Tinkturen, Shampoos, Wasch-, Dusch- oder Badezubereitungen oder Pudern vorliegen.

Als übliche kosmetische Trägerstoffe zur Herstellung der erfindungsgemäßen dermatologischen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende. Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen dermatologischen Zubereitungen, haben sich nichtionogene Typen, wie Polyoxyethylen-Fettalkoholether, z.B. Cetylstearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen dermatologischen Zubereitungen, deren pH-Wert vorzugsweise z.B. durch übliche Puffergemische auf 4,0 bis 7,5 insbesondere 5,0 bis 6,5, eingestellt wird, Parfüm, Farbstoffe, Antioxidantien, Suspendiermittel, Puffergemische oder andere übliche kosmetische oder dermatologische Grundstoffe beigemischt werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Camosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin. Glutathion, Cystein, Cystin, Cystamin und deren Glycosyl-, N-Acetyl-, Methyl-, Ethyl-Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), femer (Metall)-Chelatoren (z.B.α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist für die vorliegende Erfindung vorteilhaft, daß der pH-Wert der erfindungsgemäßen dermatologischen Zubereitungen kleiner als 8 ist. pH-Werte, die leicht höher sind als 7, aber kleiner als 7,5 können dabei im allgemeinen toleriert werden. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Vorteilhaft wird der pH-Wert der erfindungsgemäßen Formulierungen im sauren bis sehr schwach alkalischen Bereich kleiner als 8 eingestellt, bevorzugt von 4,0 - 7,5, besonders bevorzugt von 5,0 - 6,5.

Die jeweils einzusetzenden Mengen an Hilfs- Zusatz- und Trägerstoffen und gegebenenfalls Parfüm können in Abhängigkeit von der Art des jeweiligen Produktes vom Fachmann durch einfaches Ausprobieren leicht ermittelt werden.

Die Herstellung der erfindungsgemäßen dermatologischen Zubereitungen erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Sollen die erfindungsgemäßen Kombinationen in Puder eingearbeitet werden, so können die Suspensionsgrundlagen dafür vorteilhaft gewählt werden aus der Gruppe

Kieselsäuregele (z.B. solche die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die Mengenangaben beziehen sich stets auf Gewichts-%, wenn nichts Anderes angegeben wird.

### Beispiel 1

| W/O-Crème | | |
|---|---|---|
| | I | II |
| Paraffinöl | 10,00 | 10,00 |
| Ozokerit | 4,00 | 4,00 |
| Vaseline | 4,00 | 4,00 |
| pflanzliches Öl | 10,00 | 10,00 |
| Wollwachsalkohol | 2,00 | 2,00 |
| Aluminiumstearat | 0,40 | 0,40 |
| 2-Butyloctansäure | 0,10 | 0,10 |
| DMC | 0,30 | - |
| GMCy | - | 0,20 |
| Parfum, Konservierungsstoffe | .................. q.s. .................. | |
| Wasser, VES | ............. ad 100,00 ............. | |
| pH: | ............. ad 5,5 - 6,0 ........... | |

### Beispiel 2

| W/O-Lotion | | |
|---|---|---|
| | I | II |
| Paraffinöl | 25,00 | 25,00 |
| Siliconöl | 2,00 | 2,00 |
| Ceresin | 1,50 | 1,50 |
| Wollwachsalkohol | 0,50 | 0,50 |
| Glucosesesquiisostearat | 2,50 | 2,50 |
| 2-Butyloctansäure | 0,10 | - |
| 2-Butyldecansäure | - | 0,10 |
| GML | 0,50 | 0,50 |
| Parfum, Konservierungsstoffe | ................... q.s. ................... | |
| Wasser, VES | ............ ad 100,00 .............. | |
| pH: | ............. ad 5,5 - 6,0 ........... | |

### Beispiel 3

| O/W-Lotion | | |
|---|---|---|
| | I | II |
| Paraffinöl | 5,00 | 5,00 |
| Isopropylpalmitat | 5,00 | 5,00 |
| Cetylalkohol | 2,00 | 2,00 |
| Bienenwachs | 2,00 | 2,00 |
| Ceteareth-20 | 2,00 | 2,00 |
| PEG-20-Glycerylstearat | 1,50 | 1,50 |
| Glycerin | 3,00 | 3,00 |
| 2-Butyldecansäure | 0,20 | 0,20 |
| GML | 0,70 | - |
| GMM | - | 0,70 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 4

| O/W-Crème | | |
|---|---|---|
| | I | II |
| Pflanzliches Öl | 10,00 | 10,00 |
| Cetylalkohol | 2,00 | 2,00 |
| Glycerinmonostearat | 1,50 | 1,50 |
| PEG-30-Glycerylstearat | 2,00 | 2,00 |
| Glycerin | 3,00 | 3,00 |
| Isopropylpalmitat | 5,00 | 5,00 |
| Carbopol 980 (neutralisiert) | 0,30 | 0,30 |
| 2-Hexyloctansäure | 0,50 | - |
| 2-Hexyldecansäure | - | 0,50 |
| DMC | 0,30 | - |
| GMCy | 0,30 | 0,30 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 5

| Salbe | I | II |
|---|---|---|
| Vaseline | 36,00 | 36,00 |
| Ceresin | 10,00 | 10,00 |
| Zinkoxid | 4,00 | 4,00 |
| Pflanzliches Öl | 20,00 | 20,00 |
| 2-Butyldecansäure | 0,10 | - |
| 2-Hexyldecansäure | - | 0,10 |
| DMC | 0,50 | 0,50 |
| Parfum, Konservierungsstoffe | ................... q.s. ................... | |
| Paraffinöl | .............. ad 100,00 .............. | |

### Beispiel 6

| Hautöl | I | II |
|---|---|---|
| Cetylpalmitat | 3,00 | 3,00 |
| C₁₂₋₁₅- Alkylbenzoat | 2,00 | 2,00 |
| Polyisobuten | 10,00 | 10,00 |
| Squalan | 2,00 | 2,00 |
| 2-Butyloctansäure | 0,10 | - |
| 2-Butyldecansäure | - | 0,10 |
| TML | 1,00 | 1,00 |
| Parfum, Konservierungsstoffe | ................... q.s. ................... | |
| Paraffinöl | .............. ad 100,00 .............. | |

### Beispiel 7

| Badeöl | | |
|---|---|---|
| | I | II |
| Paraffinöl | 20,00 | 20,00 |
| PEG-40-hydriertes Rizinusöl | 5,00 | 5,00 |
| 2-Hexyloctansäure | 0,30 | 0,30 |
| GMC | 0,70 | - |
| GML | - | 1,00 |
| Parfum, Konservierungsstoffe | ................... q.s. ................... | |
| Sojaöl | .............. ad 100,00 .............. | |

### Beispiel 8

| Lippenstift | | |
|---|---|---|
| | I | II |
| Ceresin | 8,00 | 8,00 |
| Bienenwachs | 4,00 | 4,00 |
| Carnaubawachs | 2,00 | 2,00 |
| Vaseline | 40,00 | 40,00 |
| Hydriertes Rizinusöl | 4,00 | 4,00 |
| Caprylic/Capric Triglyceride | 6,00 | 6,00 |
| 2-Hexyldecansäure | - | 0,05 |
| 2-Hexyloctansäure | 0,05 | - |
| DML | 0,10 | 0,10 |
| Parfum, Konservierungsstoffe | ................... q.s. ................... | |
| Paraffinöl | .............. ad 100,00 .............. | |

### Beispiel 9

| Pflegemaske | | |
|---|---|---|
| | I | II |
| PEG-50 Lanolin | 0,50 | 0,50 |
| Glycerylstearat | 2,00 | 2,00 |
| Sonnenblumenkernöl | 3,00 | 3,00 |
| Bentonit | 8,00 | 8,00 |
| Kaolin | 35,00 | 35,00 |
| Zinkoxid | 5,00 | 5,00 |
| 2-Butyloctansäure | 0,10 | 0,10 |
| GMC | - | 0,20 |
| GMCy | 0,20 | - |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 10

| Liposomenhaltiges Gel | | |
|---|---|---|
| | I | II |
| Lecithin | 6,00 | 6,00 |
| Pflanzliches Öl | 12,50 | 12,50 |
| Hydrolysiertes Kollagen | 2,00 | 2,00 |
| Xanthan Gum | 1,40 | 1,40 |
| Butylenglycol | 3,00 | 3,00 |
| 2-Hexyldecansäure | 0,20 | 0,20 |
| DML | 0,50 | - |
| TMM | - | 0,50 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 11

| Duschpräparat mit Rückfetter | | |
|---|---|---|
| | I | II |
| Cocoamidodiacetat | 10,00 | 10,00 |
| Natriumlaurylsulfat | 25,00 | 25,00 |
| Kalium Cocyl Hydrolysiertes Kollagen | 5,00 | 5,00 |
| Macadamianußöl | 5,00 | 5,00 |
| Natriumchlorid | 0,60 | 0,60 |
| 2-Bintyloctansäure | 0,20 | 0,20 |
| GMCy | 0,80 | - |
| GMC | - | 0,80 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 12

| Seifenstück | | |
|---|---|---|
| | I | II |
| Na-Salz aus Talgfettsäuren | 60,00 | 60,00 |
| Na-Salz aus Kokosöl | 28,00 | 28,00 |
| Natriumchlorid | 0,50 | 0,50 |
| 2-Butyloctansäure | 0,30 | - |
| 2-Hexyldecansäure | - | 0,30 |
| GML | 0,20 | 0,20 |
| Parfum, Konservierungsstoffe | ................... q.s. ................... | |
| Wasser, VES | .............. ad 100,00 .............. | |

### Beispiel 13

| Haarpflegemittel | | |
|---|---|---|
| | I | II |
| TEA-Cocoyl hydrolysiertes Kollagen | 30,00 | 30,00 |
| Monoethanolaminlaurylsulfat | 25,00 | 25,00 |
| Mandelöl | 2,00 | 2,00 |
| Natriumchlorid | 1,00 | 1,00 |
| 2-Hexyldecansäure | - | 0,10 |
| 2-Hexyloctansäure | 0,10 | - |
| GML | 1,00 | 1,00 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 14

| Pflegeshampoo | | |
|---|---|---|
| | I | II |
| Natriumlaurylsulfat | 34,00 | 34,00 |
| Dinatriumlaurylsulfosuccinat | 6,00 | 6,00 |
| Cocoamidopropylbetain | 10,00 | 10,00 |
| Glycoldistearat | 5,00 | 5,00 |
| 2-Butylhexansäure | 0,20 | - |
| 2-Butyloctansäure | - | 0,20 |
| DMCy | 0,70 | 0,70 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 15

| Haarkur | | |
|---|---|---|
| | I | II |
| Cetylalkohol | 5,00 | 5,00 |
| Caprylic/Capric Triglyceride | 3,00 | 3,00 |
| Petrolatum | 2,00 | 2,00 |
| Wollwachsalkohol | 0,50 | 0,50 |
| 2-Hexyldecansäure | 0,20 | 0,20 |
| GMC | 0,40 | - |
| GML | - | 0,40 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 16

| Haarspülung | | |
|---|---|---|
| | I | II |
| Cocoamidopropylbetain | 5,00 | 5,00 |
| Cetylalkohol | 2,00 | 2,00 |
| Propylenglycol | 2,00 | 2,00 |
| Citronensäure | 0,30 | 0,30 |
| 2-Butyloctansäure | 0,20 | - |
| 2-Hexyloctansäure | - | 0,20 |
| GMCy | 0,50 | 0,50 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 17

| Haarfestiger | | |
|---|---|---|
| | I | II |
| Polyvinylpyrrolidon/Vinylacetat/Vinylpropionat-Copolymer | 5,00 | 5,00 |
| Ethanol | 45,00 | 45,00 |
| 2-Hexyloctansäure | 0,10 | - |
| 2-Hexyldecansäure | - | 0,10 |
| TML | 0,30 | 0,30 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 18

| Frisiercrème | | |
|---|---|---|
| | I | II |
| Vaseline | 4,00 | 4,00 |
| Cetearylalkohol | 4,00 | 4,00 |
| PEG-40-hydriertes Rizinusöl | 2,00 | 2,00 |
| Isopropylpalmitat | 5,00 | 5,00 |
| Citronensäure | 1,00 | 1,00 |
| 2-Hexyloctansäure | 0,10 | 0,10 |
| GMM | 0,30 | - |
| GMS | - | 0,20 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 19

| Rasierschaum | | |
|---|---|---|
| | I | II |
| Stearinsäure | 7,00 | 7,00 |
| Natriumlaurylsulfat | 3,00 | 3,00 |
| Stearylalkohol | 1,00 | 3,00 |
| Glycerin | 5,00 | 5,00 |
| Triethanolamin | 3,60 | 3,60 |
| 2-Butyloctansäure | 0,10 | 0,10 |
| GMC | 0,30 | - |
| GML | - | 0,30 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 20

| Fußcrème | | |
|---|---|---|
| | I | II |
| Soluan 5 | 2,00 | 2,00 |
| Methylsalicylat | 5,00 | 5,00 |
| Caprylic/Capric Triglyceride | 10,00 | 10,00 |
| Stearinsäure | 5,00 | 5,00 |
| Cetylalkohol | 1,00 | 1,00 |
| Glycerin | 2,00 | 2,00 |
| Dimethicon | 1,00 | 1,00 |
| Carbopol 984 | 0,50 | 0,50 |
| Triethanolamin | 1,50 | 1,50 |
| 2-Butylhexansäure | 0,20 | - |
| 2-Butyloctansäure | - | 0,10 |
| GMU | 0,70 | 0,70 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 21

| Aerosolspray | | |
|---|---|---|
| | I | II |
| Octyldodecanol | 0,50 | 0,50 |
| 2-Butyloctansäure | 0,10 | 0,10 |
| DMC | 0,40 | - |
| GML | - | 0,70 |
| Parfum, Konservierungsstoffe | ................... q.s. ................... | |
| Ethanol | .............. ad 100,00 .............. | |

Die durch Zusammennmischung der jeweiligen Bestandteile erhaltene flüssige Phase wird zusammen mit einem Propan-Butan-Gemisch (2:7) im Verhältnis 39:61 in Aerosolbehälter abgefüllt.

### Beispiel 22

| Pumpspray | | |
|---|---|---|
| | I | II |
| PEG-40-Hydriertes Rizinusöl | 2,00 | 2,00 |
| Glycerin | 1,00 | 1,00 |
| 2-Butyldecansäure | 0,20 | - |
| 2-Hexyldecansäure | - | 0,20 |
| GMCy | 0,40 | 0,40 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 23

| Roll-on-Gel | | |
|---|---|---|
| | I | II |
| 1,3-Butylenglycol | 2,00 | 2,00 |
| PEG-40-Hydriertes Rizinusöl | 2,00 | 2,00 |
| Hydroxyethylcellulose | 0,50 | 0,50 |
| 2-Hexyloctansäure | 0,20 | - |
| 2-Hexyldecansäure | - | 0,30 |
| DMC | 0,50 | 0,50 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 24

| Roll-on-Emulsion | | |
|---|---|---|
| | I | II |
| Tricetearethphosphat | 0,30 | 0,30 |
| Octyldodecanol | 2,00 | 2,00 |
| C₁₂₋₁₅-Alkylbenzoat | 2,00 | 2,00 |
| C₁₀₋₃₀-Alkylacrylat | 0,15 | 0,15 |
| 2-Hexyloctansäure | 0,30 | 0,30 |
| GMC | 0,60 | - |
| GML | - | 0,80 |
| Parfum, Konservierungsstoffe | ................... q.s. ................... | |
| Wasser, VES | .............. ad 100,00 .............. | |
| pH: | ............. ad 5,5 - 6,0 ........... | |

### Beispiel 25

| Wachsstift | | |
|---|---|---|
| | I | II |
| Hydriertes Rizinusöl | 5,00 | 5,00 |
| Bienenwachs | 6,00 | 6,00 |
| Ceresin | 30,00 | 30,00 |
| C₁₂₋₁₅-Alkylbenzoat | 17,00 | 17,00 |
| 2-Butylhexansäure | 0,20 | - |
| 2-Butyloctansäure | - | 0,20 |
| TML | 0,70 | 0,70 |
| Parfum, Konservierungsstoffe | ................... q.s. ................... | |
| Octyldodecanol | .............. ad 100,00 .............. | |

## Patentansprüche

1. Kosmetische Verwendung von Wirkstoffkombinationen, bestehend aus
(I) einer oder mehrerer Substanzen, gewählt aus der Gruppe der Monoglycerin-monocarbonsäureester, der Diglycerin-monocarbonsäureester, der Triglycerin-monocarbonsäureester, der Monoglycerin-dicarbonsäure-monoester, der Diglycerin-dicarbonsäuremonoester und der Triglycerin-dicarbonsäure-monoester sowie
(II) einer oder mehreren dialkylsubstituierten Essigsäuren der Formel 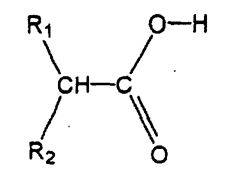
wobei R1 einen verzweigten oder unverzweigten Alkylrest mit 1 - 12 Kohlenstoffatomen und R2 einen verzweigten oder unverzweigten Alkylrest mit 1 - 24 Kohlenstoffatomen darstellt,
zur Bekämpfung von Körpergerüchen.

2. Verwendung von Wirkstoffkombinationen, bestehend aus
(I) einer oder mehrerer Substanzen, gewählt aus der Gruppe der Monoglycerin-monocarbonsäureester, der Diglycerin-monocarbonsäureester, der Triglycerin-monocarbonsäureester, der Monoglycerin-dicarbonsäure-monoester, der Diglycerin-dicarbonsäuremonoester und der Triglycerin-dicarbonsäure-monoester sowie
(II) einer oder mehreren dialkylsubstituierten Essigsäuren der Formel 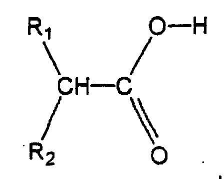
wobei R1 einen verzweigten oder unverzweigten Alkylrest mit 1 - 12 Kohlenstoffatomen und R2 einen verzweigten oder unverzweigten Alkylrest mit 1 - 24 Kohlenstoffatomen darstellt,
zur Herstellung dermatologischer Zubereitungen zur Bekämpfung von Körpergerüchen.

3. Kosmetische Verwendung von Wirkstoffkombinationen, bestehend aus
(I) einer oder mehrerer Substanzen, gewählt aus der Gruppe der Monoglycerin-monocarbonsäureester, der Diglycerin-monocarbonsäureester, der Triglycerin-monocarbonsäureester, der Monoglycerin-dicarbonsäure-monoester, der Diglycerin-dicarbonsäuremonoester und der Triglycerin-dicarbonsäure-monoester sowie
(II) einer oder mehreren dialkylsubstituierten Essigsäuren der Formel 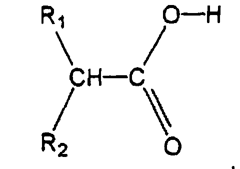
wobei R1 einen verzweigten oder unverzweigten Alkylrest mit 1 - 12 Kohlenstoffatomen und R2 einen verzweigten oder unverzweigten Alkylrest mit 1 - 24 Kohlenstoffatomen darstellt,
zur Bekämpfung von Kopfschuppen.

4. Verwendung von Wirkstoffkombinationen, bestehend aus
(I) einer oder mehrerer Substanzen, gewählt aus der Gruppe der Monoglycerin-monocarbonsäureester, der Diglycerin-monocarbonsäureester, der Triglycerin-monocarbonsäureester, der Monoglycerin-dicarbonsäure-monoester, der Diglycerin-dicarbonsäure-monoester und der Triglycerin-dicarbonsäure-monoester sowie
(II) einer oder mehreren dialkylsubstituierten Essigsäuren der Formel 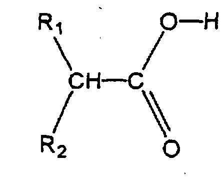
wobei R1 einen verzweigten oder unverzweigten Alkylrest mit 1 - 12 Kohlenstoffatomen und R2 einen verzweigten oder unverzweigten Alkylrest mit 1 - 24 Kohlenstoffatomen darstellt,
zur Herstellung dermatologischer Zubereitungen zur Bekämpfung von Kopfschuppen.

5. Verwendung nach einem der verhergehenden Ansprüche, **dadurch gekennzeichnet, daß** die Substanz oder die Substanzen der Gruppe der Monoglycerin-monocarbonsäure-mono-ester, der Diglycerin-monocarbonsäure-monoester und der Triglycerin-monocarbonsäuremonoester gewählt wird aus der Gruppe Glycerinmonocaprylat, Glycerinmonocaprinat, Diglycerinmonocaprinat, Triglycerinmonolaurat, Diglycerinmonolaurat, Triglycerinmonomyristat.

6. Verwendung nach einem der verhergehenden Ansprüche **dadurch gekennzeichnet, daß** als dialkylsubstituierte Essigsäure die 2-Butyloctansäure gewählt wird.

## Claims

1. Cosmetic use of active compound combinations comprising
(I) one or more substances chosen from the group consisting of monoglycerol monocarboxylic acid esters, diglycerol monocarboxylic acid esters, triglycerol monocarboxylic acid esters, monoglycerol dicarboxylic acid monoesters, diglycerol dicarboxylic acid monoesters and triglycerol dicarboxylic acid monoesters and
(II) one or more dialkyl-substituted acetic acids of the formula 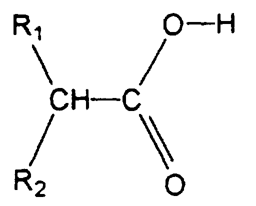
wherein R1 is a branched or unbranched alkyl radical having 1 - 12 carbon atoms and R2 is a branched or unbranched alkyl radical having 1 - 24 carbon atoms, for combating body odours.

2. Use of active compound combinations comprising
(I) one or more substances chosen from the group consisting of monoglycerol monocarboxylic acid esters, diglycerol monocarboxylic acid esters, triglycerol monocarboxylic acid esters, monoglycerol dicarboxylic acid monoesters, diglycerol dicarboxylic acid monoesters and triglycerol dicarboxylic acid monoesters and
(II) one or more dialkyl-substituted acetic acids of the formula 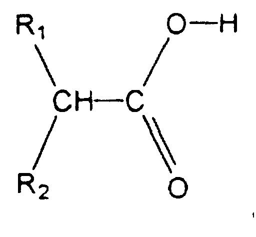
wherein R1 is a branched or unbranched alkyl radical having 1 - 12 carbon atoms and R2 is a branched or unbranched alkyl radical having 1 - 24 carbon atoms for the preparation of dermatological formulations for combating body odours.

3. Cosmetic use of active compound combinations comprising
(I) one or more substances chosen from the group consisting of monoglycerol monocarboxylic acid esters, diglycerol monocarboxylic acid esters, triglycerol monocarboxylic acid esters, monoglycerol dicarboxylic acid monoesters, diglycerol dicarboxylic acid monoesters and triglycerol dicarboxylic acid monoesters and
(II) one or more dialkyl-substituted acetic acids of the formula 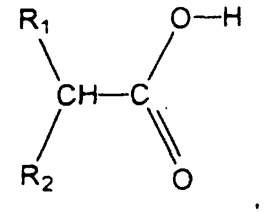
wherein R1 is a branched or unbranched alkyl radical having 1 - 12 carbon atoms and R2 is a branched or unbranched alkyl radical having 1 - 24 carbon atoms for the preparation of dermatological formulations for combating dandruff.

4. Use of active compound combinations comprising
(I) one or more substances chosen from the group consisting of monoglycerol monocarboxylic acid esters, diglycerol monocarboxylic acid esters, triglycerol monocarboxylic acid esters, monoglycerol dicarboxylic acid monoesters, diglycerol dicarboxylic acid monoesters and triglycerol dicarboxylic acid monoesters and
(II) one or more dialkyl-substituted acetic acids of the formula 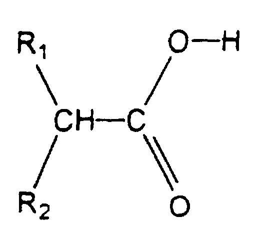
wherein R1 is a branched or unbranched alkyl radical having 1 - 12 carbon atoms and R2 is a branched or unbranched alkyl radical having 1 - 24 carbon atoms for the preparation of dermatological formulations for combating dandruff.

5. Use according to one of the preceding claims, **characterized in that** the substance or the substances of the group consisting of monoglycerol monocarboxylic acid monoesters, diglycerol monocarboxylic acid monoesters and triglycerol monocarboxylic acid monoesters are chosen from the group consisting of glycerol monocaprylate, glycerol monocaprate, diglycerol monocaprate, triglycerol monolaurate, diglycerol monolaurate and triglycerol monomyristate.

6. Use according to one of the preceding claims, **characterized in that** 2-butyloctanoic acid is chosen as the dialkyl-substituted acetic acid.

## Revendications

1. Utilisation cosmétiques d'associations de substances actives, constituées de
(I) une ou plusieurs substances choisies dans le groupe des esters d'acides monocarboxyliques avec le monoglycérol, des esters d'acides monocarboxyliques avec le diglycérol, des esters d'acides monocarboxyliques avec le triglycérol, des monoesters d'acides dicarboxyliques avec le monoglycérol, des monoesters d'acides dicarboxyliques avec le diglycérol et des monoesters d'acides dicarboxyliques avec le triglycérol, ainsi que
(II) un ou plusieurs acides acétiques disubstitués par des groupes alkyle, de formule 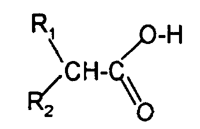 dans laquelle R₁ représente un radical alkyle ramifié ou non ramifié ayant de 1 à 12 atomes de carbone, et R₂ représente un radical alkyle ramifié ou non ramifié ayant de 1 à 24 atomes de carbone,
pour combattre des odeurs corporelles.

2. Utilisation d'associations de substances actives, constituées de
(I) une ou plusieurs substances choisies dans le groupe des esters d'acides monocarboxyliques avec le monoglycérol, des esters d'acides monocarboxyliques avec le diglycérol, des esters d'acides monocarboxyliques avec le triglycérol, des monoesters d'acides dicarboxyliques avec le monoglycérol, des monoesters d'acides dicarboxyliques avec le diglycérol et des monoesters d'acides dicarboxyliques avec le triglycérol, ainsi que
(II) un ou plusieurs acides acétiques disubstitués par des groupes alkyle, de formule 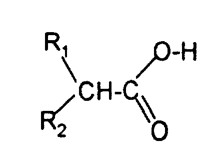 dans laquelle R₁ représente un radical alkyle ramifié ou non ramifié ayant de 1 à 12 atomes de carbone, et R₂ représente un radical alkyle ramifié ou non ramifié ayant de 1 à 24 atomes de carbone,
pour la fabrication de préparations dermatologiques destinées à combattre les odeurs corporelles.

3. Utilisation cosmétique d'associations de substances actives, constituées de
(I) une ou plusieurs substances choisies dans le groupe des esters d'acides monocarboxyliques avec le monoglycérol, des esters d'acides monocarboxyliques avec le diglycérol, des esters d'acides monocarboxyliques avec le triglycérol, des monoesters d'acides dicarboxyliques avec le monoglycérol, des monoesters d'acides dicarboxyliques avec le diglycérol et des monoesters d'acides dicarboxyliques avec le triglycérol, ainsi que
(II) un ou plusieurs acides acétiques disubstitués par des groupes alkyle, de formule 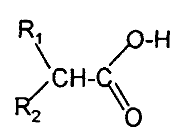 dans laquelle R₁ représente un radical alkyle ramifié ou non ramifié ayant de 1 à 12 atomes de carbone, et R₂ représente un radical alkyle ramifié ou non ramifié ayant de 1 à 24 atomes de carbone,
pour combattre les pellicules.

4. Utilisation d'associations de substances actives, constituées de
(I) une ou plusieurs substances choisies dans le groupe des esters d'acides monocarboxyliques avec le monoglycérol, des esters d'acides monocarboxyliques avec le diglycérol, des esters d'acides monocarboxyliques avec le triglycérol, des monoesters d'acides dicarboxyliques avec le monoglycérol, des monoesters d'acides dicarboxyliques avec le diglycérol et des monoesters d'acides dicarboxyliques avec le triglycérol, ainsi que
(II) un ou plusieurs acides acétiques disubstitués par des groupes alkyle, de formule 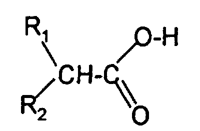 dans laquelle R₁ représente un radical alkyle ramifié ou non ramifié ayant de 1 à 12 atomes de carbone, et R₂ représente un radical alkyle ramifié ou non ramifié ayant de 1 à 24 atomes de carbone,
pour la fabrication de préparations dermatologiques destinées à combattre les pellicules.

5. Utilisation selon l'une quelconque des revendications précédentes,
**caractérisée en ce que** la substance ou les substances du groupe des monoesters d'acides monocarboxyliques avec le monoglycérol, des monoesters d'acides monocarboxyliques avec le diglycérol et des monoesters d'acides monocarboxyliques avec le triglycérol est(sont) choisie(s) dans le groupe constitué par le monocaprylate de glycérol, le monocaprate de glycérol, le monocaprate de diglycérol, le monolaurate de triglycérol, le monolaurate de diglycérol, le monomyristate de triglycérol.

6. Utilisation selon l'une quelconque des revendications précédentes,
**caractérisée en ce que** l'acide 2-butyloctanoïque est choisi en tant qu'acide acétique disubstitué par des groupes alkyle.
